# EUROPEAN PATENT SPECIFICATION

(11) **EP 0 930 861 B1**
(45) Date of publication and mention of the grant of the patent: **16.01.2002**
(21) Application number: 96936330.8
(22) Date of filing: 11.10.1996
(51) Int. Cl.: A61F 13/15, B32B 31/00

(54) **COVERSHEET COMPRISING NONWOVEN AND APERTURED FILM**
DECKLAGE AUS VLIESSTOFF UND PERFORIERTER FOLIE
FEUILLE DE COUVERTURE COMPRENANT UN NON-TISSE ET UN FILM AJOURE

(43) Date of publication of application: 28.07.1999
(73) Proprietor: THE PROCTER & GAMBLE COMPANY, Cincinnati, Ohio 45202 (US)
(72) Inventor: CHATTERJEE, Ashish, Higashinada-ku, Kobe 658 (JP)
(74) Representative: Hirsch, Uwe Thomas
(86) International application number: US9616239
(87) International publication number: WO9816177

(56) References cited:
- EP-A- 0 403 187
- EP-A- 0 596 532
- WO-A-93/09741
- GB-A- 2 284 786
- US-A- 4 781 962
- US-A- 4 919 738

## Description

### FIELD OF THE INVENTION

This invention relates to coversheets for absorbent articles such as diapers, incontinent articles, sanitary napkins, and the like. More particularly, this invention relates to a coversheet for absorbent articles comprising a nonwoven and an apertured thermoplastic film. This invention further relates to methods of laminating plural layers, at least one of which is thermoplastic film or web or nonwoven or the like.

### BACKGROUND OF THE INVENTION

All manner and variety of absorbent articles configured for the absorption of body fluids are well known. Current types of absorbent articles include diapers, incontinent articles, and sanitary napkins.

A major in use problem encountered with known absorbent articles is leakage of waste product which contaminates clothing articles that contact the absorbent article, such as pants, shirts, and bedding. The amount of leakage experienced by the wearer can be reduced by improving the rate at which the liquid enters the absorbent core. Thus, an absorbent article in which the liquid rapidly penetrates the topsheet and is contained in the absorbent core will experience less leakage than an absorbent article in which liquid is able to run across the topsheet before penetrating into the absorbent core. Reducing run-off, therefore, reduces the amount of leakage experienced with the absorbent article.

Another in-use problem associated with absorbent articles is the dryness of the skin contacting surface. Generally, the drier the skin contacting surface, the more comfortable the absorbent article. There have been several patents directed towards reducing the surface wetness in disposable diaper structures. U.S. Patent 3,945,386 issued to Anczurowski on March 23, 1976 and U.S. Patents 3,965,906 and 3,994,299 issued to Karami on June 29, 1976 and November 30, 1976, respectively, teach diaper structures having a perforated thermoplastic film interposed between the topsheet and the absorbent core. U.S. Patent 4,324,247 issued to Aziz on April 13, 1982 describes an effort directed to both reducing run-off and reducing the surface wetness of absorbent articles. WO 93/09741 published on May 27, 1993 describes a laminated topsheet comprising a nonwoven and an apertured film which is treated with a surfactant to enhance the overall permeability of the topsheet.

U.S. Patent 3,530,023 issued to Schutte et al. on September 22, 1970 describes "Laminated Sheet Material And Methods Of Making Such Material". As disclosed, such material comprises at least two adjacent layers of cellulosic fiber sheet material, and a layer of thermoplastic material which layers are secured together at a plurality of bonding areas by heat and pressure without the addition of any adhesive material. This patent states that such bonding may be achieved by forwarding the layers through a nip between two rolls which rolls are arranged to maintain a fixed special relationship relative to each other. As further disclosed, one of the rolls may be smooth surfaced with the other having spaced projections extending outwardly from its cylindrical surface; or both rolls may have such projections.

U.S. Patent 4,035,219 issued to Cumbers on July 12, 1977 describes a nonwoven structure, method and apparatus for producing nonwoven. In this apparatus as disclosed, a thermoplastic non-woven is first formed as by extruding the thermoplastic from a spinneret; and then passing the filamentary mass through bonding means. In the bonding means, a bonding member such as a roll is provided which has projections on it; the bonding member is heated to a temperature below the softening point of the thermoplastic to be bonded, and the bonding member is pressure biased towards a backing member such as a roll; and the material to be bonded is passed there between. For example, through the nip between a pair of pressure biased nip rollers: a heated pattern roller having projections; and a backing roller. Bonding is said to be effected by virtue of the work done by the pressure biased, heated projections to compress the material.

U.S. Patent 4,854,984 issued to Ball et al. on August 8, 1989 and U.S. Patent 4,919,738 issued to Ball et al. on April 24, 1990 describe "Dynamic Mechanical Bonding Method and Apparatus". The disclosed method and apparatus for dynamically mechanically bonding together a plurality of laminae, at least one of which comprises thermoplastic material, for example, polyethylene. In one aspect of the invention which is particularly useful at intermediate and higher line velocities - preferably for line velocities of about 300 feet or more per minute and, more preferably, for line speeds of about 450 feet or more per minute - the nip defining members may be operated with equal surface velocities. The members may be biased towards each other to provide a predetermined pattern-element-psi loading.

EP-A-596 532 discloses a film laminated material and process and apparatus for making same. The laminate comprises a film and non-woven material, both comprising polypropylene. However the preferred film comprises 62% polypropylene disregarding the disadvantages associated with such high polypropylene content as explained supra.

While prior art laminated sheets, prior art laminating apparatuses and methods for laminated sheets together have addressed some of the problems of achieving such lamination, they have not addressed the problems to the extent of or in the manner of the present invention.

### SUMMARY OF THE INVENTION

This invention relates to coversheets for absorbent articles such as diapers, incontinent articles, sanitary napkins, and the like. More particularly, this invention relates to a coversheet for absorbent articles comprising a nonwoven and an apertured thermoplastic film. This invention further relates to methods of laminating plural layers, at least one of which is thermoplastic film or web or nonwoven or the like.

A coversheet for an absorbent article is disclosed. The coversheet comprises at least a nonwoven and an apertured thermoplastic film with face to face relation which are joined at least by applying pressure. The nonwoven comprises at least polypropylene. The apertured thermoplastic film comprises from more than 0 % to less than about 30 % of polypropylene and from more than about 70 % to less than 100 % of polyethylene.

A method of bonding plural layers together with face to face relation is disclosed. The method comprises: forwarding layers comprising a nonwoven and an apertured thermoplastic film, the nonwoven comprising at least polypropylene and the apertured thermoplastic film comprising from more than 0 % to less than about 30 % of polypropylene and from more than about 70 % to less than 100 % of polyethylene, at a predetermined velocity through a pressure biased nip between a patterned nip defining member having pattern element segments, and a nip defining anvil member; biasing said nip defining members towards each other with a predetermined loading.

### BRIEF DESCRIPTION OF THE DRAWINGS

Figure 1 is a perspective view of an absorbent article using a coversheet of the present invention.
Figure 2 is a cross-sectional view taken along line II-II of Figure 1.
Figure 3 is an edge view of an apertured thermoplastic film comprising part of a coversheet of the present invention.
Figure 4 is an edge view of an embodiment of a completely assembled coversheet.
Figure 5 is a somewhat schematic, fragmentary side elevational view of an apparatus embodiment of the present invention.
Figure 6 is a perspective view of a patterned cylinder of a bonding apparatus shown in FIG. 5.
Figure 7 is an enlarged scale, fragmentary view looking inwardly toward a pattern element which is disposed on a cylindrical surface of a patterned cylinder shown in FIG. 6.
Figure 8 is a cross-fragmentary sectional view taken along line VIII-VIII of FIG. 7.
Figure 9 is an enlarged scale, fragmentary plan view of two layers bonded together through the use of the present invention.

### DESCRIPTION OF THE PREFERRED EMBODIMENTS

This invention relates to coversheets for absorbent articles such as diapers, incontinent articles, sanitary napkins, and the like. More particularly, this invention relates to a coversheet for absorbent articles comprising a nonwoven and an apertured thermoplastic film. This invention further relates to methods of laminating plural layers, at least one of which is thermoplastic film or web or nonwoven or the like.

The term "absorbent article", as used herein, refers to articles which absorb and contain body exudates. More specifically, the term refers to articles which are placed against or in proximity to the body of the wearer to absorb and contain the various exudates discharged from the body. The term "absorbent article" is intended to include diapers, incontinent articles, sanitary napkins, pantiliners, and other articles used to absorb body exudates. The term "disposable" refers to articles which are intended to be discarded after a single use and preferably recycled, composted, or otherwise disposed of in an preferably recycled, composted, or otherwise disposed of in an environmentally compatible manner. (That is, they are not intended to be laundered or otherwise restored or reused as an absorbent article.)

The term "diaper" refers to a garment generally worn by infants and incontinent persons which is drawn up between the legs and fastened about the waist of the wearer. Suitable diapers that can be provided with the coversheet described herein are disclosed in U.S. Patent Re. 26,152, issued to Duncan, et al. on January 31, 1967; U.S. Patent 3,860,003 issued to Buell on January 14, 1975; U.S. Patent 4,610,678 issued to Weisman, et al. on September 9, 1986; U.S. Patent 4,673,402 issued to Weisman, et al. on June 16, 1987; U.S. Patent 4,695,278 issued to Lawson on September 22, 1987; U.S. Patent 4,704,115 issued to Buell on November 3, 1987; U.S. patent 4,834,735 issued to Alemany, et al. on May 30, 1989; U.S. patent 4,888,231 issued to Angstadt on December 19, 1989; and U.S. Patent 4,909, 803 issued to Aziz, et al. on March, 1990.

The term "incontinent article" refers to pads, undergarments (pads held in place by a suspension system of same type, such as a belt, or the like), inserts for absorbent articles, capacity boosters for absorbent articles, briefs, bed pads, and the like regardless of whether they are worn by adults or other incontinent persons. Suitable incontinent articles that can be provided with the coversheet described herein are disclosed in U.S. Patent 4,253,461 issued to Strickland, et al. on March 3, 1981; U.S. patents 4,597,760 and 4,597,761 issued to Buell; the above-mentioned U.S. Patent 4,704,115; U.S. Patent 4,909,802 issued to Ahr, et al.; U.S. Patent 4,964,860 issued to Gipson et al. on October 23, 1990; and in U.S. Patent Application Serial Numbers 07/637,090 and 07/637,571 filed respectively by Noel, et al. and Feist, et al. on January 3, 1991.

The term "sanitary napkin" refers to an article which is worn by females adjacent to the pudendal region that is intended to absorb and contain various exudates which are discharged from the body (e.g., blood, menses, and urine). Suitable sanitary napkins that can be provided with the coversheet described herein are disclosed in U.S. patent 4,285,343, issued to McNair on August 25, 1981; U.S, Patents 4,589,876 and 4.687,478, issued to Van Tilburg on May 20, 1986 and August 18, 1987 respectively; U.S. Patents 4,917,697 and 5,007,906 issued to Osborn, et al. on April 17, 1990 and April 16, 1991, respectively; and U.S. patents 4,950,264 and 5,009,653 issued to Osborn on August 21, 1990 and April 23, 1991, respectively; and in U.S. Patents Application Serial No. 07/605,583 filed October 29, 1990 in the name of Visscher, et al.

The term "pantiliner" refers to absorbent articles that are less bulky than sanitary napkins which are generally worn by women between their menstrual periods. Suitable pantiliners that can be provided with the coversheet described herein are disclosed in U.S. Patent 4,738,676 entitled " Pantiliner" issued to Osborn on April 19,1988.

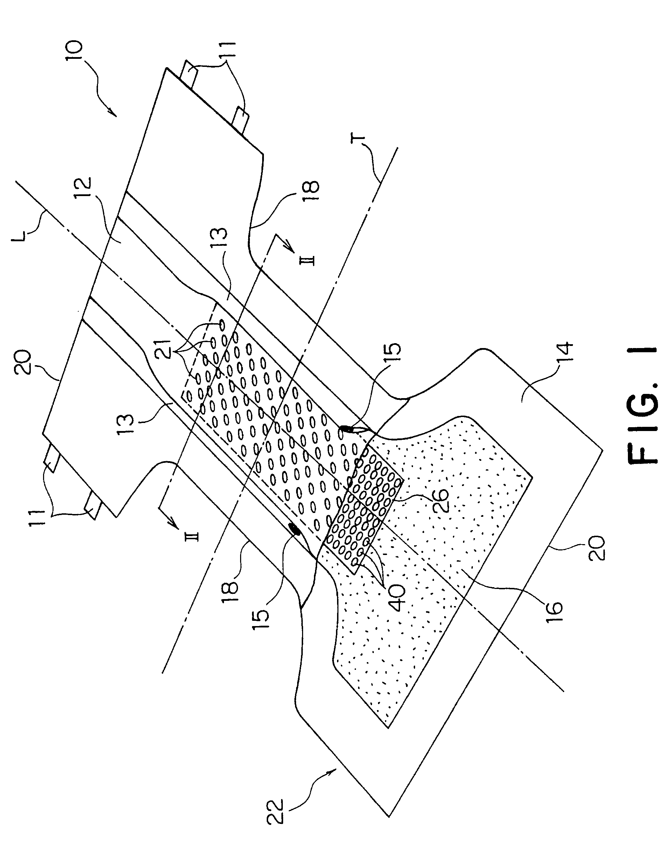

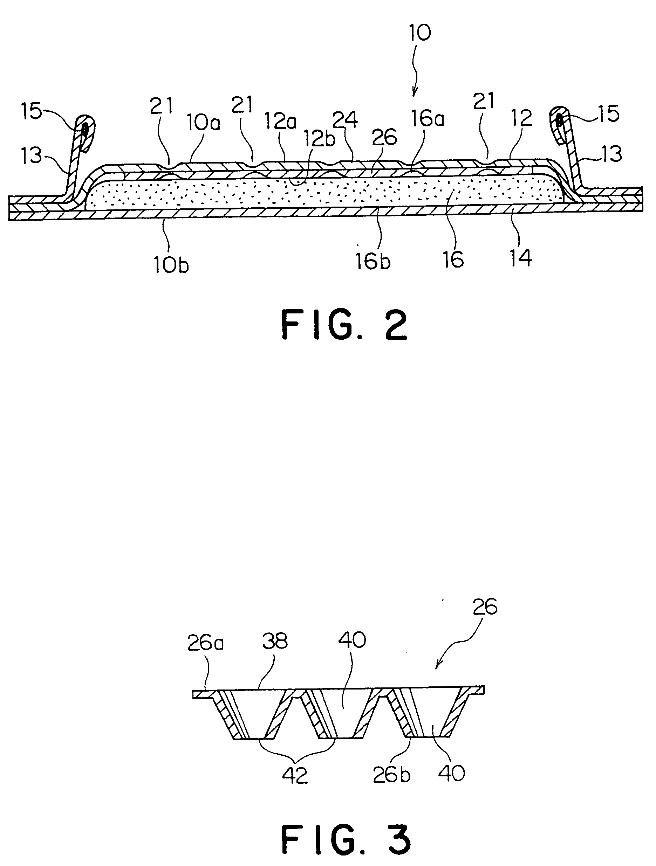

Figure 1 shows a simplified absorbent article 10 that could represent a diaper prior to its being placed on a wearer. It should be understood, however, that the present invention is not limited to the particular type or configuration of absorbent article shown in the drawings. As shown in figure 2, such an absorbent article 10 basically comprises topsheet 12, backsheet 14, and absorbent core 16. The absorbent article 10 further comprises fastener means 11 for securing the back side and the front side of the absorbent article 10. Barrier leg cuffs 13 are provided onto the topsheet 12 to prevent body exudates from leaking out. Means 15 for standing the barrier leg cuffs 13 such as elastics may be provided at the edge of the cuffs 13. The topsheet 12 comprises two layers that are a nonwoven 24 and a three dimensional apertured thermoplastic film 26. In Fig. 1, a part of the nonwoven 24 is removed to show the three dimensional apertured thermoplastic film 26 and the absorbent core 16.

The absorbent article 10 has two surfaces, a body-facing surface (or "body surface") 10a and a garment facing surface (or "garment surface") 10b. The body surface 10a is intended to be worn adjacent to the body of the wearer. The garment surface 10b of the absorbent article 10 (shown in FIG. 2) is on the opposite side and is intended to be placed adjacent to the wearer's undergarments or clothing when the absorbent article 10 is worn.

The absorbent article 10 has two centerlines, a longitudinal centerline L and a transverse centerline T. The terms "longitudinal" and "transverse" or "lateral" (the latter two being interchangeable), are defined in U.S. Patent 5,007,906 issued to Osborn, III, et al. and are applicable to the absorbent article 10 described herein. Figure 1 shows that the absorbent article described herein. Figure 1 shows that the absorbent article 10 has two spaced apart longitudinal edges 18 and two spaced apart transverse or end edges (or "ends") 20, which together form the periphery 22 of the absorbent article 10. The individual components of the absorbent article 10 will now be looked at in greater detail.

The topsheet 12 is compliant, soft-feeling and non-irritating to the wearer's skin. Further, the topsheet 12 is liquid permeable, permitting liquids to readily penetrate through its thickness. The topsheet 12 has a body-facing side or face 12a and a garment-facing side or face 12b. (A similar numbering system will be used for the other components of the diaper 10. That is, the side of the component facing the wearer's body will be designated by the number of the component and a reference letter "a", the side facing the wearer's undergarments by the number of the component and the letter "b" respectively.)

The topsheet 12 comprises two components, a nonwoven material, preferably in the form of fabric 24, and the three dimensional apertured thermoplastic film 26. The nonwoven 24 has the same shape as the backsheet 14. The apertured thermoplastic film 26 is rectangle and relatively smaller than the nonwoven 24. The apertured thermoplastic film 26 positions at the middle of both longitudinal direction and the transverse direction. In other words, the apertured thermoplastic film 26 generally covers the region where body exudates come. The apertured thermoplastic film 26 may extend between both end edges 18 or may have any size. The nonwoven 24 and the apertured thermoplastic film 26 are joined to each other at many discrete points 21 by applying pressure and/or heat. In the embodiment shown in Fig. 1, the nonwoven 24 positions to face a wearer's body and the apertured thermoplastic film 26 positions to face the absorbent core 16. The component parts of the topsheet 12 are examined in greater detail below.

The nonwoven 24 may be any nonwoven fabric that is permeable to liquids. A suitable nonwoven 24 may be manufactured from wide range of materials. The nonwoven 24 preferably comprises at least polypropylene. The nonwoven 24 may be made from a mixture of polypropylene and fibers from a group consisting of polypropylene, polyester, polyethylene, polyvinylalcohol, starch base resins, polyurethanes, cellulose and cellulose esters. Preferably, the nonwoven 24 is made from a mixture of from more than about 25 % to less than or equal to 100 % of polypropylene and from more than or equal to 0 % to less than about 75 % of polyethylene. More preferably, the nonwoven 24 is made from a mixture of from more than about 50 % to less than or equal to 100 % of polypropylene and from more than or equal to 0 % to less than about 50 % of polyethylene.

Clearly, there are a number of manufacturing techniques which may be utilized to manufacture the nonwoven 24. For example, the nonwoven 24 may be resin-bonded, needle punched, spunbonded, carded, the latter including, thermally bonded, air-through bonded, and spunlaced fabrics. A preferred nonwoven 24 is a thermally bonded fabric.

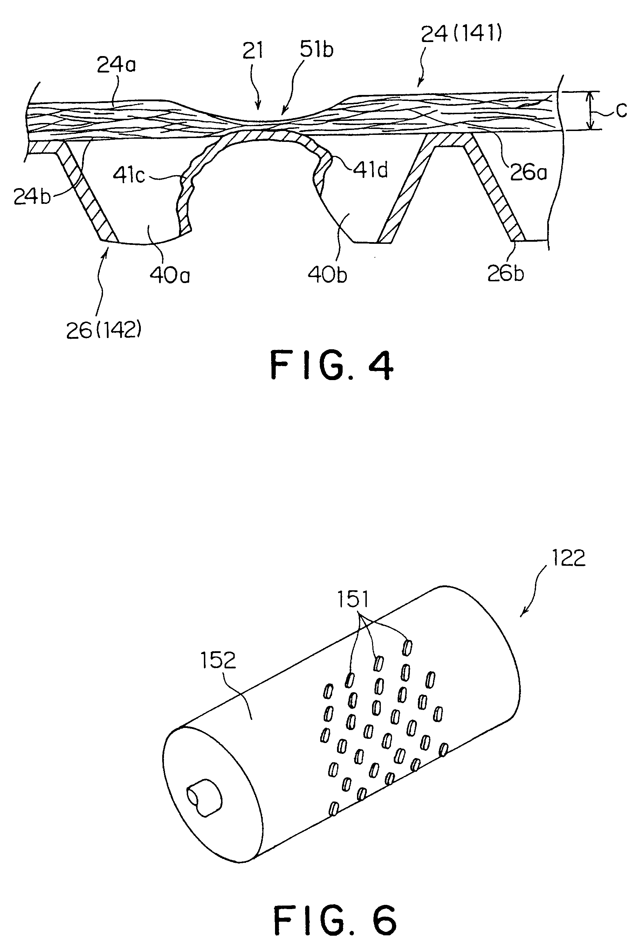

The nonwoven 24 should be lightweight having a weight from about 1 to about 40 g/sq.m, preferably from about 1 to about 23 g/sq.m. For one embodiment, the nonwoven 24 has a basis weight range of from about 15 to about 22 grams per square yard (about 18 to about 26 g/sq.m.) and a caliper C as shown in Figure 4, of from about 5 to about 15 mils (about 0.13 to about 0.38 mm.) when measured under a load of about 200 pascals. Such a nonwoven 24 is further characterized by a minimum wet or dry tensile strength of at least about 400 grams per centimeter in the longitudinal or machine direction and at least about 55 grams per centimeter in the cross machine direction.

The three dimensional apertured thermoplastic film 26 is preferably located between the nonwoven 24 and the absorbent core 16. As shown in Figure 3, the apertured thermoplastic film 26 is preferably a three-dimensional structure being capable of temporarily containing body exudates such as body liquids before body liquids are absorbed by the absorbent core 16. The apertured thermoplastic film 26 has a plurality of tapered capillaries 40, each of which has a base opening 38, and an apex opening 42. The apex openings 42 are preferably in intimate contact with the absorbent core 16.

The three dimensional apertured thermoplastic film 26 is manufactured from a liquid impervious, preferably thermoplastic material. One suitable material comprises a low density polyethylene film having a thickness of from 0.001 to 0.002 inches (0.0020 to 0.0051 cm.). The thermoplastic material for use in the manufacture of the apertured thermoplastic film 26 preferably comprises from more than 0 % to less than about 30 % of polypropylene and from more than about 70 % to less than 100 % of polyethylene. More preferably, the apertured thermoplastic film 26 comprises from more than 0 % to less than about 20 % of polypropylene and from more than about 80 % to less than 100 % of polyethylene. The thermoplastic material used in the present invention preferably has a density in the range of from about 0.90 g/cm³ to about 1.20 g/cm³, with the more preferred range of densities being from about 0.91 g/cm3 to about 0.99 g/cm³. The general melt indices range for such material is preferably from about 2 to about 100, with the more preferred range being from about 4 to about 25.

In one preferred embodiment, the three dimensional thermoplastic material is provided with a multiplicity of tapered capillaries in a manner, size, configuration, and orientation set forth in U.S. Patent 3,939,135 issued to Thompson on December 30, 1975. Other suitable apertured plastic films are disclosed in U.S. Patent 4,324,426, issued to Mullane, et al. on April 13, 1982, U.S. Patent 4,342,314, issued to Radel, et al. on August 3, 1982, and U.S. Patent 4,463,045, issued to Ahr, et al. on July 31, 1984.

The nonwoven 24 and the apertured thermoplastic film 26 may be placed into a face-to-face relationship. The two components may be secured. The two components may be secured to each other by many different methods (or securement means) or combinations of methods. Suitable methods for securing the two components include, but are not limited to adhesives, fusion including heat bonding and/or pressure bonding, ultrasonics, and dynamic mechanical bonding. A preferable method is described hereinafter.

The absorbent core 16 is positioned between the topsheet 12 and the backsheet 14. The absorbent core 16 provides the means for absorbing bodily exudates. The absorbent core 16 need not have an absorbent capacity much greater than the total amount of exudates to be absorbed. The absorbent core 16 is generally compressible, conformable, and non-irritating to the user's skin. It can comprise any material used in the art for such purpose. Examples include comminuted wood pulp which is generally referred to as airfelt, creped cellulose wadding, cross-linked cellulose fibers, absorbent foams, absorbent sponges, synthetic staple fibers, polymeric fibers, hydrogel-forming polymer gelling agents, peat moss, combinations of the foregoing, or any equivalent material or combinations of materials.

Suitable cross-linked cellulose fibers are described in U.S. Patent 4,888,093, issued December 19, 1989 to Cook, et al.; U.S. Patent 4,822,543, issued April 18, 1989 to Dean, et al.; U.S. Patent 4,889,595, issued December 26, 1989 to Schoggen, et al.; U.S. Patent 4,889,596, issued December 26, 1989 to Schoggen, et al.; U.S. Patent 4,898,642, issued February 6, 1990 to Moore, et al.; and U.S. Patent 4,935,022, issued June 19, 1990 Lash, et al.

The characteristics of the absorbent core 16 for particular types of absorbent articles are described in greater detail in the patents and documents incorporated by reference herein, and the patents and other documents incorporated by reference in those documents, the disclosures of which are all incorporated by reference herein. Other suitable absorbent core arrangements are described in U.S. Patents 4,988,344 and 4,988,345, and European Patent Application Publication No. 0 198 683, published October 22, 1986 in the name of Duenk, et al. which are also incorporated by reference herein. The absorbent article 10 could also include any additional layers or other components such as are described in the patents incorporated by reference. For example, the absorbent article 10 may comprise an acquisition layer or patch of cross-linked cellulose fibers positioned between the topsheet 12 and the absorbent core 16.

The backsheet 14 is impervious to liquids and is preferably manufactured from a thin plastic film, although other flexible liquid impervious materials may also be used. The backsheet 14 prevents liquid contained in absorbent core 16 from wetting articles which contact the absorbent article 10. Polyethylene films having a thickness of from about 0.001 to about 0.002 inches (0.0025 to 0.0051 cm.) have been used for the backsheet 14 with satisfactory results. As used herein, the term "flexible" refers to materials which are compliant and which will readily conform to the general shape and contours of the human body.

The backsheet 14 is superimposed on the garment-facing side 16b of absorbent core 16 and preferably extends beyond the edges thereof. The topsheet 12 is superimposed over the body-facing side 16a of the absorbent core 16, and may also extend beyond the edges of the core 16. The absorbent core 16 is, therefore, positioned between the topsheet 12 and the backsheet 14. The topsheet 12 and backsheet 14 are joined to each other such as around their peripheries. The topsheet 12 and backsheet 14 can be joined in any suitable manner such as by the use of adhesives, crimping, heat-sealing, or ultrasonic bonding. A more detailed description of how topsheet 12, backsheet 14, and absorbent core 16 may be assembled for particular types of absorbent articles is provided in the documents incorporated by reference herein.

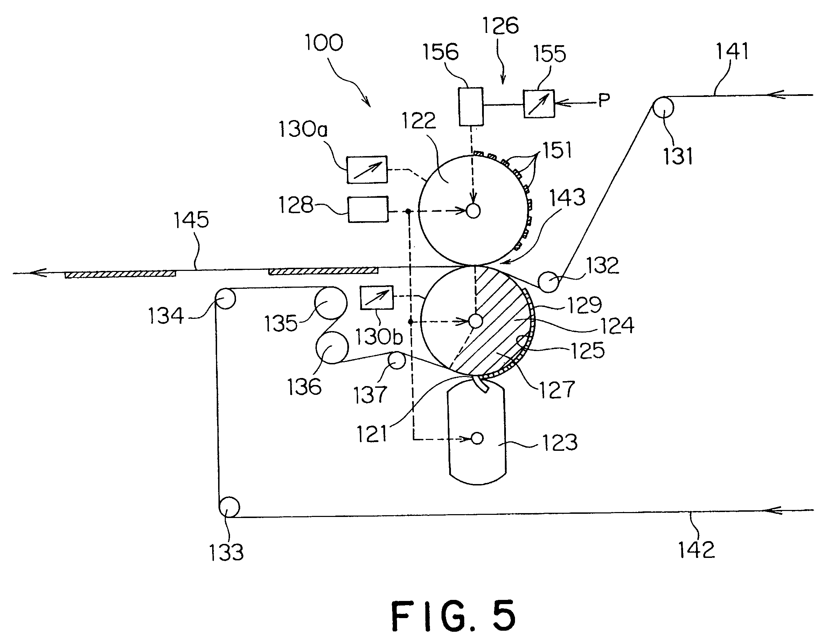

A suitable process of preparing the topsheet 12 is shown in Figure 5. In Figure 5, a somewhat schematic, fragmentary side elevational view of a dynamic mechanical bonding apparatus 100 which is an embodiment of the present invention is shown. Apparatus 100 comprises a patterned cylinder 122, an anvil cylinder 124, a blade cylinder 123, means 126 for adjustably biasing the cylinders 122 and 124 towards each other with a predetermined pressure within a predetermined range of pressures, means 128 for rotating the cylinders 122 and 124 at controlled velocities to provide a predetermined surface velocity, temperature control means 130a and 130b for heating the cylinders 122 and 124 to provide predetermined surface temperature thereon, and rolls 131 through 137. A nonwoven web 141, an apertured thermoplastic film web 142, and a laminae 145 being joined both webs 141 and 142, are also shown in Fig. 5.

For clarity of the present invention, neither the upstream ends or sources of the nonwoven web 141 and the apertured thermoplastic film web 142, nor the downstream destination or user of the laminae 145 are shown. However, for example, it is well known to provide web of thermoplastic films, and paper and other web in roll form; and to provide upstream unwinding and splicing means to enable forwarding continuous lengths of such a web through laminating means and or converters to make products comprising laminated and/or other web elements at controlled velocities and under controlled tension.

Parenthetically, for simplicity and clarity of the invention, the apparatus 100 is described herein as comprising the cylinders 122 and 124. However, the cylinders are but exemplary nip defining members as stated hereinbefore. Accordingly, it is not intended to thereby limit the invention to apparatus comprising cylinders per se. In the same vein, use of the term pattern element is not intended to limit the invention to bonding patterns consisting of only discrete, spaced pattern elements to the exclusion of other patterns: e.g., reticulated patterns or patterns comprising continuous or elongate lines of bonding.

Briefly, referring to the apparatus 100, Figure 5, the present invention enables thermolaminating certain laminae together - providing the nonwoven web comprises at least polypropylene comprising sufficient thermoplastic material that is susceptible to being thermobonded to the apertured thermoplastic web comprising from more than 0 % to less than about 30 % of polypropylene and from more than about 70 % to less than 100 % of polyethylene - by forwarding the webs together through a pressure biased nip between a patterned cylinder and an anvil cylinder which cylinders have a predetermined surface velocity. Such laminating can be effected at substantially lower nip biasing pressure (i.e., substantially lower psi loadings on the pattern elements of the patterned cylinder) with high bonding strength between both webs because both webs comprise the same chemical component of polypropylene. Directionally, the greater the ratio of polypropylene in the apertured thermoplastic web, the lower the required nip biasing pressure and the higher the bonding strength between both webs. However, too great a ratio of polypropylene in the apertured thermoplastic web may result in making the apertured thermoplastic film manufacturing process difficult, for example, in the process of using heat and vacuum as stated in, for example, U.S. Patent 4,601,868, issued July 22, 1986 to Radel, et al. The softening point, which makes the material soft and is lower than the melting point, of polyethylene is lower than that of polypropylene and polyethylene is softer than polypropylene. Therefore, the higher polyethylene ratio in the apertured thermoplastic film allows to use a low temperature and low vacuum in the process using heat and vacuum. Therefore, from more than 0 % to less than about 30 % of the polypropylene ratio in the apertured thermoplastic film is preferred to achieve enough bonding strength and easy manufacturing process of the apertured thermoplastic film. It also allows manufacturing process operations at reduced pressure. This reduces dust which is usually formed as a by-product of the bonding process using high pressure. Therefore, dust build-up on the cylinders 122 and 124 can be reduced. As a result, this allows the process to operate without any process aids such as wax to prevent dust build-up. In addition, such reduced pressure allows long life of pattern of the patterned cylinder. Further, such laminating may be effected with even lower nip biasing pressure if one or both of the cylinders is heated: preferably to temperatures which are sufficiently lower than the melting points of the laminae that the laminae will not melt or stick to the laminating cylinders in the event, for example, the apparatus is temporarily stopped.

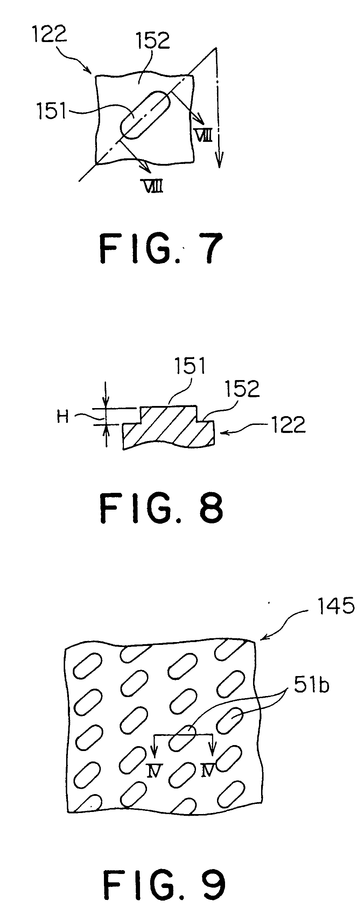

Referring now to Figure 5, the patterned cylinder 122 is configured to have a circular cylindrical surface 152, and a plurality of protuberances or pattern elements 151 which extend outwardly from surface 152. The protuberances 151 are disposed in a predetermined pattern: each pattern element being configured and disposed to precipitate a bond site in the laminate being produced to effect a predetermined pattern of bond sites in the laminate. As shown in Figure 6, the cylinder 122 has a discrete dotted shape pattern of protuberances 151 which extends circumferentially in a part of the cylinder 122.

Anvil cylinder 124, Figure 5, is preferably a smooth surfaced, right circular cylinder of steel. In an exemplary embodiment of the invention, the anvil cylinder 124 has a 4.5 inch (about 11.4 cm.) diameter. The anvil cylinder 124 has a surface rotating member 125 which rotates independently from the inside structure of the anvil cylinder 124. The surface rotating member 125 is structured to rotate together with the patterned cylinder 124 by drive means described hereinafter. The anvil cylinder 124 is provided with a vacuum means 127 inside in the region shown by hatching in Figure 5. The vacuum means 127 holds a cut web 129 on the surface rotating means 125.

Blade cylinder 123, Figure 5, has a blade 121 on the surface The blade 121 has enough length along the perpendicular direction to the direction where web 142 is conveyed, to cut web 142 to each cut web 129. The blade cylinder 123 rotates with synchronized the anvil cylinder 124 and cuts web 142 in cooperation with anvil (not shown) provided on the surface of the anvil cylinder 124.

Means 126, Figure 5, for biasing patterned cylinder 122 towards the anvil cylinder 124 comprises pressure regulating means 155, and pneumatic actuator means 56. Pressure regulating means 155 is adapted to have its inlet connected to a supply source P of pressurized air, and to have its outlet connected to pneumatic actuator means 156 in order to adjust and control the pneumatic actuator means loading of cylinders 122 and 124 towards each other. Whereas only one pneumatic actuator or means 156 is visible in Figure 5, identical actuators are in fact connected to each end journal of the cylinder; and, of course, each end journal is supported by frame members and ancillary hardware (not shown) to be vertically moveable so that, in fact, the pressure biasing means can be effective.

Drive means 128 in Figure 5 is provided to drive cylinders 122, 124 and 123. Thus, it constitutes means for power rotating the cylinders so that there is a predetermined relationship betweenthere surface velocities. In an exemplary embodiment that is integrated into a disposable diaper converter, cylinders 122, 124 and 123 are driven by a direct current motor. Alternatively, they may be driven by a converter line drive through a gear train so that the surface velocities are essentially matched to the line velocity of the converter. Each cylinders 122 and 124 may have different means for power rotating cylinders respectively so that each cylinders can be controlled independently to have a predetermined surface velocity differential with either cylinder being driven faster than the other. This enables adjusting the surface velocity of the anvil cylinder to be equal to, or less than, or greater than the surface velocity of the patterned cylinder by predetermined amounts or percentages.

Temperature control means 130a and 130b, Figure 5, may be provided to adjustable control the surface temperatures of the cylinders 122 and 124, respectively. Alternatively, either of the temperature control means 130a and 130b may be eliminated. The apparatus 100 may have only the temperature control means 130a for heating the patterned cylinder 122. As stated above, these means enable independently heating each of the cylinders 122 and 124 to establish surface temperatures thereon that are predetermined degrees below the melt temperature of the thermoplastic web disposed most adjacent to each. Such heating enables effecting thermobonding of the laminae at lower nip biasing pressure than would otherwise be required for any given line speed and surface velocity differential between the cylinders 122 and 124; and obviates having the laminae melting and sticking to the cylinders during, for example, converter and/or laminator stops.

Rolls 131 through 137 are provided for guiding and advancing webs 141 and 142, and laminae 145 through nip 143. Preferably these rolls are driven at surface velocities which maintain predetermined levels of tension or stretch so that neither slack web conditions nor excessively tensioned/stretched webs and or laminate precipitate undesirable deleterious consequences. For example, in an exemplary disposable diaper converter comprising the present invention, rolls 131 and 132, and cylinders 122, 123 and 124 are driven at the same velocity. Rolls 133 through 137 are driven slower than the rolls 131 and 132, and cylinders 122, 123 and 124. Therefore, web 142 is provided to the surface of the anvil cylinder 124 at slower velocity than the velocity at which the surface rotating means 125 rotates. While cut web 129 is conveyed at the velocity of the surface rotating means 125 being faster than the velocity of web 142, web 142 which is not cut yet is conveyed at the velocity controlled by the rolls 133 through 137. Thereby cut webs 129 are provided with a certain interval. Cut webs 129 are intermittently joined to continuous web 141.

Turning now to Figure. 7, a fragmentary portion of cylinder 122 is shown which comprises one pattern element 151 disposed on the cylindrical surface 152. Figure 8, a fragmentary sectional view taken along section line 8-8 of Figure 7, shows that the pattern element 151 is an integral portion of the cylinder 122, has substantially vertical side surfaces, and projects radically outwardly a distance H: i.e., the radial height of the pattern element. While such an integral relationship is preferred, it is not intended to thereby limit the present invention to such integral constructions. In an exemplary apparatus 100, pattern element 151 has an oval planform having a width of about 0.059 inch (about 1.5 mm.), length of about 0.079 inch (about 2 mm.), end radii of about 0.030 inch (about 0.75 mm), and are oriented on the surface of cylinder 122 with their length dimensions extending at 45 degrees with the circumferential direction. Starting with a right circular cylinder, pattern elements 151 were machined by removing surrounding metal by electric discharge machining to a depth of from about 0.015 to about 0.020 inch (about 0.4 mm to about 0.5 mm). Additionally, they were spaced - center to center - about 0.138 inch (about 3.5 mm) circumferentially (i.e., in the machine direction), and about 0.276 (about 7 mm) inch laterally (i.e., in the cross machine direction).

Figure 9 is a plan view of a fragmentary portion of laminate 145, Figure 5, comprising webs 41 and 42 which have been thermobonded together by a pattern of pattern elements which extends circumferentially in a part of the cylinder 122.

Figure 4, is a somewhat schematic, fragmentary sectional view taken along section line 4-4 of Figure 9, which illustratively shows a bond site 51b which thermobonds webs 141 (nonwoven 24) and 142 (apertured thermoplastic film 26) together to form laminate 145. The bond site 51b shown in Figure 4 is formed at lease by applying pressure between one of the protuberances 151 and the surface rotating member 125. As shown in Figure 4, a right hand side wall 41c of the tapered capillary 40a and a left hand side wall 41d of the tapered capillary 40b are deformed by applying pressure. Nonwoven 24 is also deformed by pressure.

The apertured thermoplastic film 26 comprising from more than 0 % to less than about 30 % of polypropylene and from more than about 70 % to less than 100 % of polyethylene, and the nonwoven 24 comprising at least polypropylene which are joined at least by applying pressure exhibits many benefits. The attachment by using pressure of the apertured thermoplastic film 26 having more than 0 % of polypropylene and the nonwoven 24 comprising at least polypropylene shows an enhanced bonding strength, while the apertured thermoplastic film 26 having less than about 30 % of polypropylene allows easy manufacturing process of making the apertured thermoplastic film 26 (i.e. using a relatively low temperature and a relatively low vacuum). Such an enhanced bonding strength allows to use relatively low pressure and relatively high process velocity in making a coversheet comprising two layers joined at least by applying pressure, compared with the pressure and velocity used conventionally. Pressure may be from more than about 60,000 psi to less than about 100,000 psi. Preferably, it may be from more than about 60,000 psi to less than about 75,000 psi. Pressure of more than about 60,000 psi ensures enough bonding strength. Pressure of less than about 75,000 psi ensures reduced dust that is a by-product of the process. Velocity may be more than about 250 feet per minute, preferably more than about 450 feet per minute. More preferably, it may be from more than about 550 to less than about 700 feet per minute. The apertured thermoplastic film comprising from more than 0 % to less than 30 % also account for the manufacturing process dealing with a patch design film that is attached to the nonwoven intermittently. In the manufacturing process dealing with a patch design film that is conveyed at a certain interval given after the film is cut as described in Figure 5, the apertured thermoplastic patch design film 29 reduces snap-back when the film is cut because polypropylene which is stiffer than polyethylene is contained.

Bond strength data was measured at 600 feet per minute of cylinder surface velocity, room temperature (i.e. cylinder surface was unheated), and a bonding pressure of 60,000 - 63,000 psi. Bond strength data for coversheet of a nonwoven comprising 100 % of polypropylene and an apertured thermoplastic film comprising 15 % of polypropylene and 85 % of polyethylene was about double as strong as bond strength data for coversheet of a nonwoven comprising 100 % of polypropylene and an apertured thermoplastic film comprising 100 % of polyethylene. Also, by-product dust in the bonding process was significantly reduced.

It will be understood by those skilled in the art that the invention has been described with reference to an exemplary preferred embodiment and that variations and modifications can be effected in the described embodiment without departing from the scope and spirit of the invention.

## Claims

1. A coversheet (12) for an absorbent article (10), the coversheet (12) comprising a nonwoven (24) and an apertured thermoplastic film (26) with face to face relation, the nonwoven (24) and the apertured thermoplastic (26) being joined at least by applying pressure,
the nonwoven (24) comprises at least polypropylene and said coversheet being **characterized in that** the apertured thermoplastic film (26) comprises from more than 0 % to less than about 30 % of polypropylene and from more than about 70 % to less than 100 % of polyethylene.

2. The coversheet (12) of Claim 1 wherein the apertured thermoplastic film (26) comprises from more than 0 % to less than about 20 % of polypropylene and from more than about 80 % to less than 100 % of polyethylene.

3. The coversheet (12) of Claim 1 wherein the nonwoven (24) comprises from more than about 25 % to less than or equal to 100 % of polypropylene and from more than or equal to 0 % to less than about 75 % of polyethylene.

4. The coversheet (12) of Claim 2 wherein the absorbent article (10) comprises an absorbent core (16), wherein the nonwoven (24) is positioned to face a wearer body and the apertured thermoplastic film (26) is positioned to be adjacent to the absorbent core (16).

5. The coversheet (12) of Claim 4 wherein the nonwoven (24) and the apertured thermoplastic film (26) are joined at many discrete points by applying pressure.

6. The coversheet (12) of Claim 1 wherein the apertured thermoplastic film (26) is shorter in the machine direction than the absorbent article (10).

7. The coversheet (12) of Claim 6 wherein the apertured thermoplastic film (26) is shorter in the machine direction than the nonwoven (24).

8. A method of bonding plural layers together with face to face relation, the method comprising of:
forwarding layers comprising a nonwoven (24) and an apertured thermoplastic film (26), the nonwoven (24) comprising at least polypropylene and the apertured thermoplastic film (26) comprising from more than 0 % to less than about 30 % of polypropylene and from more than about 70 % to less than 100 % of polyethylene, at a predetermined velocity through a pressure biased nip (143) between a patterned nip defining member (122) having pattern element (151) segments. and a nip defining anvil member (124),
biasing said nip defining members (122, 124) towards each other with a predetermined loading.

9. The method of Claim 8 wherein the steps further comprises biasing said nip defining members (122, 124) towards each other with a predetermined loading from more than about 60,000 psi to less than about 75,000 psi.

10. The method of Claim 8 wherein the steps further comprises: cutting the apertured thermoplastic film in the machine direction; giving intervals between cut films; and providing the cut films to the nonwoven at intervals before the layers are forwarded to the pressure biased nip (143).

## Patentansprüche

1. Ein Deckblatt (12) für einen absorbierenden Artikel (10), wobei das Deckblatt (12) ein Faservlies (24) und eine mit Öffnungen versehene thermoplastische Folie (26) mit Fläche-an-Fläche-Beziehung umfaßt, wobei das Faservlies (24) und die mit Öffnungen versehene thermoplastische Folie (26) mindestens durch Aufbringen von Druck verbunden sind,
wobei das Faservlies (24) mindestens Polypropylen umfaßt und das genannte Deckblatt **dadurch gekennzeichnet ist, daß** die genannte mit Öffnungen versehene thermoplastische Folie (26) von mehr als 0 % bis weniger als etwa 30 % Polypropylen und von mehr als etwa 70 % bis weniger als etwa 100 % Polyethylen umfaßt.

2. Das Deckblatt (12) nach Anspruch 1, bei welchem die mit Öffnungen versehene thermoplastische Folie (26) von mehr als 0 % bis weniger als etwa 20 % Polypropylen und von mehr als etwa 80 % bis weniger als 100 % Polyethylen umfaßt,

3. Das Deckblatt (12) nach Anspruch 1, bei welchem das Faservlies (24) von mehr als etwa 25 % bis weniger als oder gleich 100 % Polypropylen und von mehr als oder gleich 0 % bis weniger als etwa 75 % Polyethylen umfaßt.

4. Das Deckblatt (12) nach Anspruch 2, wobei der absorbierende Artikel (10) einen absorbierenden Kern (16) umfaßt, wobei das Faservlies (24) positioniert ist, um einem Körper eines Trägers zugewandt zu sein, und die mit Öffnungen versehene thermoplastische Folie (26) positioniert ist, um anliegend an den absorbierenden Kern (16) zu sein.

5. Das Deckblatt (12) nach Anspruch 4, bei welchem das Faservlies (24) und die mit Öffnungen versehene thermoplastische Folie (26) an vielen diskreten Punkten durch Aufbringen von Druck verbunden sind.

6. Das Deckblatt (12) nach Anspruch 1, bei welchem die mit Öffnungen versehene thermoplastische Folie (26) in der Mäschinenrichtung kürzer ist als der absorbierende Artikel (10).

7. Das Deckblatt (12) nach Anspruch 6, bei welchem die mit Öffnungen versehene thermoplastische Folie (26) in der Maschinenrichtung kürzer ist als das Faservlies (24).

8. Ein Verfahren zum Aneinanderbinden von mehreren Schichten mit Fläche-an-Fläche-Beziehung, wobei das Verfahren umfaßt:
Weiterleiten von Schichten, welche ein Faservlies (24) und eine mit Öffnungen versehene thermoplastische Folie (26) umfassen, wobei das Faservlies (24) mindestens Polypropylen umfaßt und die mit Öffnungen versehene thermoplastische Folie (26) von mehr als 0 % bis weniger als etwa 30 % Polypropylen und von mehr als etwa 70 % bis weniger als 100 % Polyethylen umfaßt, bei einer festgelegten Geschwindigkeit durch einen mit Druck beaufschlagten Walzenspalt (143) zwischen einem gemusterten Walzenspalt-definierenden Bauteil (122) mit Musterelement-Segmenten (151) und einem Walzenspalt-definierenden Amboßbauteil (124),
Beaufschlagen der genannten Walzenspalt-definierenden Bauteile (122, 124) gegeneinander mit einer festgelegten Last.

9. Das Verfahren nach Anspruch 8, bei welchem die Schritte weiters das Beaufschlagen der genannten Walzenspalt-definierenden Bauteile (122, 124) gegeneinander mit einer festgelegten Last von mehr als etwa 60,000 psi bis weniger als etwa 75 000 psi umfaßt.

10. Das Verfahren nach Anspruch 8, bei welchem die Schritte weiters umfassen:
Schneiden der mit Öffnungen versehenen thermoplastischen Folie in der Maschinenrichtung; Verleihen von Abständen zwischen geschnittenen Folien; und Beigeben der geschnittenen Folien dem Faservlies in Abständen, bevor die Schichten zum mit Druck beaufschlagten Walzenspalt (143) weitergeleitet werden.

## Revendications

1. Feuille de couverture (12) pour article absorbant (10), la feuille de couverture (12) comprenant un non-tissé (24) et un film thermoplastique ajouré (26), face contre face, le non-tissé (24) et le thermoplastique ajouré (26) étant reliés au moins sous l'action d'une pression,
le non-tissé (24) comprend au moins du polypropylène et ladite feuille de couverture étant **caractérisée en ce que** le film thermoplastique ajouré (26) comprend de plus de 0% à moins d'environ 30% de polypropylène et de plus d'environ 70% à moins de 100% de polyéthylène.

2. Feuille de couverture (12) selon la revendication 1 dans laquelle le film thermoplastique ajouré (26) comprend de plus de 0% à moins d'environ 20% de polypropylène et de plus d'environ 80% à moins de 100% de polyéthylène.

3. Feuille de couverture (12) selon la revendication 1 dans laquelle le non-tissé (24) comprend de plus d'environ 25% à moins de ou à 100% de polypropylène et de plus de ou de 0% à moins d'environ 75% de polyéthylène.

4. Feuille de couverture (12) selon la revendication 2 dans laquelle l'article absorbant (10) comprend une âme absorbante (16), dans laquelle le non-tissé (24) est placé contre le corps d'un utilisateur et le film thermoplastique ajouré (26) est placé en position adjacente à l'âme absorbante (16).

5. Feuille de couverture (12) selon la revendication 4 dans laquelle le non-tissé (24) et le film thermoplastique ajouré (26) sont reliés en de nombreux points discrets sous l'action d'une pression.

6. Feuille de couverture (12) selon la revendication 1 dans laquelle le film thermoplastique ajouré (26) est plus court dans le sens machine que l'article absorbant (10).

7. Feuille de couverture (12) selon la revendication 6 dans laquelle le film thermoplastique ajouré (26) est plus court dans le sens machine que le non-tissé (24).

8. Procédé pour joindre plusieurs couches ensemble face contre face, le procédé comprenant les étapes consistant à:
faire avancer des couches comprenant un non-tissé (24) et un film thermoplastique ajouré (26), le non-tissé (24) comprenant au moins du polypropylène et le film thermoplastique ajouré (26) comprenant de plus de 0% à moins d'environ 30% de polypropylène et de plus d'environ 70% à moins de 100% de polyéthylène, à une vitesse prédéterminée à travers une zone de pincement appuyée sous pression (143) entre un élément définissant une zone de pincement à motifs (122) comportant des segments d'éléments de motifs (151) et un élément d'enclume (124) définissant la zone de pincement,
appuyer lesdits éléments définissant la zone de pincement (122, 124) l'un vers l'autre avec une charge prédéterminée.

9. Procédé selon la revendication 8 dans lequel les étapes comprennent par ailleurs le fait d'appuyer lesdits éléments définissant la zone de pincement (122, 124) l'un vers l'autre avec une charge prédéterminée de plus d'environ 60 000 psi à moins d'environ 75 000 psi.

10. Procédé selon la revendication 8 dans lequel les étapes comprennent aussi le fait de découper le film thermoplastique ajouré dans le sens machine; de prévoir des intervalles entre les films découpés; et d'amener les films découpés sur le non-tissé à intervalles avant que les couches soient avancées vers la zone de pincement appuyée sous pression (143).
